# EUROPEAN PATENT APPLICATION

(11) **EP 2 997 982 A1**
(43) Date of publication of application: **23.03.2016**
(21) Application number: 14797907.4
(22) Date of filing: 07.05.2014
(51) Int. Cl.: A61L 15/64, A61K 31/765, A61K 31/77, A61P 41/00, C08G 63/664, C08L 67/04

(54) **SUBSTANCE FOR PREVENTING ADHESION AND PRODUCTION METHOD THEREFOR**

(30) Priority: 13.05.2013 JP 2013101388
(71) Applicant: A School Corporation Kansai University, Suita-shi, Osaka 564-8680 (JP); Kawasumi Laboratories, Inc., Saiki-shi, Oita 876-0121 (JP)
(72) Inventor: OHYA, Yuichi, Suita-shi Osaka 564-8680 (JP); YOSHIDA, Yasuyuki, Suita-shi Osaka 564-8680 (JP); TAKAHASHI, Akihiro, Suita-shi Osaka 564-8680 (JP); MUKAI, Tomokazu, Bungo-Ono-shi Oita 879-7153 (JP); KANEHIRA, Koji, Bungo-Ono-shi Oita 879-7153 (JP)
(74) Representative: Held, Stephan
(86) International application number: PCT/JP2014/062236
(87) International publication number: WO 2014/185309

(57) **Abstract**

The present invention relates to an adhesion-preventing material comprising the following (1) and (2): (1) an A-B-A type or B-A-B type triblock copolymer that has an A segment containing poly(ε-caprolactone-co-glycolic acid) and a B segment containing polyethylene glycol; and (2) polyethylene glycol and/or methoxy-polyethylene glycol. The present invention provides an adhesion-preventing material that undergoes a sol-gel transition in response to temperature, that can be prepared before use more easily than before, that can be easily applied to invasion sites having a complicated shape or structure, that can exhibit an adhesion-preventing effect stably for a desired period of time, and that has excellent handling properties in laparoscopic surgery.

## Description

### Technical Field

The present invention relates to an adhesion-preventing material and a method for producing the same.

### Background Art

Adhesion is a phenomenon of bonding between invasion sites or between an invasion site and its surrounding tissue resulting from, for example, invasion to the surface of body tissues (also called wound or tissue damage), bleeding, inflammation, etc., during surgery. The formation of adhesion not only induces pain in patients, but also disturbs the normal function of the organs, thus problematically causing postoperative complications.

For example, in the abdominal cavity after surgery, adhesion between the intestinal tract and the abdominal wall, or between the intestinal tracts may cause intestinal obstruction (ileus). In this case, further surgery is required, which imposes mental and physical burdens on patients. This is thus a major issue.

Therefore, in order to prevent adhesion, various adhesion-preventing materials have been conventionally developed using biocompatible and biodegradable materials. Currently used adhesion-preventing materials are widely used to prevent adhesion by physically blocking or separating invasion sites from other body tissues until the tissues of the invasion sites are repaired or healed.

For example, as adhesion-preventing materials having biocompatibility and biodegradability, PTL 1 and PTL 2 propose adhesion-preventing materials mainly comprising polylactic acid, polyglycolic acid, polycaprolactone (or a lactic acid/glycolic acid copolymer, a lactic acid/glycolic acid/ε-caprolactone copolymer, or a binary or ternary polymer of these monomers), etc. These materials are mainly used in the form of films (sheets).

Use examples of these film-like adhesion-preventing materials are as follows. After a digestive organ, such as esophagus, stomach, small intestine, large intestine, or rectum, is operated by laparoscopic surgery, the film-like adhesion-preventing material is rounded into a cylindrical shape, and inserted into the abdominal cavity from a port (a tube for inserting/ejecting a treatment instrument). Further, after gastrectomy, hepatectomy, inguinal hernia, ovariectomy, uterine myomectomy, or the like is performed by laparotomy, the film-like adhesion-preventing material is inserted from the incision site, and attached or closely anchored to the invasion site.

However, when the film-like adhesion-preventing materials are used for invasion sites having a complicated shape or in laparoscope, the conventional adhesion-preventing materials induce various problems. For example, when a film is closely anchored to an invasion site having a complicated shape, the film is broken or folded. Thus, the operability is not necessarily good. There is another problem in that even if the film-like adhesion-preventing material can be successfully attached to the invasion site, the adhesion-preventing material is shifted later from the invasion site because of the patient's action.

On the other hand, in consideration of the ease of application to invasion sites, a gel-like adhesion-preventing material is applied in place of attaching the film-like adhesion-preventing material. To enhance the effect of the adhesion-preventing material, it is necessary to retain the adhesion-preventing material in the body tissues for a certain period of time. For this reason, the concentration and viscosity of gel-like adhesion-preventing materials are increased to improve the retention of the materials, thereby enhancing the adhesion-preventing effect (see, for example, PTL 3).

However, when such a gel-like adhesion-preventing material having a high concentration and viscosity is applied to a desired invasion site from a narrow port in laparoscopic surgery, it is hard to apply the highly concentrated and viscous gel-like material. Thus, there is difficulty in handling.

In recent years, many studies have been focused on physicochemical responses of stimuli-responsive polymers to changes in temperature, pH, electric field, and chemical substance. In particular, temperature-responsive polymers that exhibit a phase transition phenomenon in response to external temperature changes have been widely studied as medical-use materials, such as drug carriers.

NPL 1 reports a triblock copolymer comprising a copolymer of poly(ε-caprolactone) (PCL) and polyglycolic acid (PGA) (hereinafter referred to as "PCGA"), and polyethylene glycol (PEG) (hereinafter referred to as "PCGA-b-PEG-b-PCGA" or simply as "PCGA-PEG-PCGA"), wherein the triblock copolymer is biodegradable and is gelled in response to temperature *in situ.* The PCGA-b-PEG-b-PCGA undergoes a sol-gel transition in response to temperature, and is solid at ordinary temperature and in the absence of solvent.

However, to dissolve the PCGA-b-PEG-b-PCGA in water or a water-containing medium, such as phosphate-buffered saline (PBS), to thereby obtain a uniformly dissolved aqueous solution, it is problematically necessary to heat it to a temperature higher than the melting point of the PCGA-b-PEG-b-PCGA. This causes another problem in that it is difficult to immediately use the PCGA-b-PEG-b-PCGA by dissolving it when needed in medical sites, such as hospitals and clinics; that is, preparation before use is difficult.

Accordingly, there is a demand for development of adhesion-preventing materials that can solve the above problems.

### Citation List

### Patent Literature

PTL 1: JP2001-192337A
PTL 2: JPH04-283227A
PTL 3: JP2010-035744A

### Non-patent Literature

NPL 1: Lin Yu et al., Macromolecular Research, 21, 207-215 (2013)

### Summary of Invention

### Technical Problem

An object of the present invention is to provide an adhesion-preventing material that undergoes a sol-gel transition in response to temperature, that can be prepared before use more easily than before, that can be easily applied to invasion sites having a complicated shape or structure, that can exhibit an adhesion-preventing effect stably for a desired period of time, that has excellent handling properties in laparoscopic surgery, and that is highly biologically safe; and also to provide a method for producing the same.

### Solution to Problem

As a result of extensive studies to achieve the above object, the present inventors found that the object can be achieved by adding polyethylene glycol and/or methoxy-polyethylene glycol to a specific A-B-A type or B-A-B type triblock copolymer. Thus, the present invention has been completed.
1. An adhesion-preventing material comprising the following (1) and (2):
   (1) an A-B-A type or B-A-B type triblock copolymer that has an A segment containing poly(ε-caprolactone-co-glycolic acid) and a B segment containing polyethylene glycol; and
   (2) polyethylene glycol and/or methoxy-polyethylene glycol.
2. The adhesion-preventing material according to item 1, wherein the polyethylene glycol and/or methoxy-polyethylene glycol (2) is contained in an amount of 2 to 20 parts by mass based on 100 parts by mass of the triblock copolymer (1).
3. The adhesion-preventing material according to item 1 or 2, wherein the A segment constitutes 30 to 75 mass% of the triblock copolymer (1), and the B segment constitutes 25 to 70 mass% of the triblock copolymer (1).
4. A method for producing an adhesion-preventing material comprising the following (1) and (2):
   (1) an A-B-A type or B-A-B type triblock copolymer that has an A segment containing poly(ε-caprolactone-co-glycolic acid) and a B segment containing polyethylene glycol; and
   (2) polyethylene glycol and/or methoxy-polyethylene glycol;
   the method sequentially comprising the following (i) and (ii):
   (i) step 1 of polymerizing ε-caprolactone and glycolide in the presence of polyethylene glycol, methoxy-polyethylene glycol, or aliphatic diol to produce the triblock copolymer (1); and
   (ii) step 2 of dissolving the triblock copolymer (1) and the polyethylene glycol and/or methoxy-polyethylene glycol (2) in a solvent.
5. The method for producing an adhesion-preventing material according to item 4, the method further comprising step 3 of drying the adhesion-preventing material obtained in step 2 to a solid state.

### 1. Adhesion-Preventing Material

The adhesion-preventing material of the present invention comprises the following (1) and (2):
(1) an A-B-A type or B-A-B type triblock copolymer that has an A segment containing poly(ε-caprolactone-co-glycolic acid) and a B segment containing polyethylene glycol; and
(2) polyethylene glycol and/or methoxy-polyethylene glycol.

Therefore, the adhesion-preventing material of the present invention undergoes a sol-gel transition in response to temperature. In particular, the adhesion-preventing material of the present invention has a feature of being gelable at a temperature between room temperature and body temperature.

The adhesion-preventing material of the present invention can be dissolved in water or a water-containing medium for a short period of time without the need to be heated (e.g., at room temperature). Therefore, the material has excellent operability and can be used immediately when needed by dissolving it.

The adhesion-preventing material of the present invention is in a liquid form when applied, in contrast to film-like (sheet-like) adhesion-preventing materials. Therefore, the material can be easily applied to invasion sites having a complicated shape or structure.

After application, the adhesion-preventing material of the present invention becomes a gel due to the body temperature. Therefore, the material can be blocked and separated from other body tissues for a certain period of time, and thus can exhibit an adhesion-preventing effect stably for a desired period of time.

The adhesion-preventing material of the present invention is in a liquid form when applied, in contrast to gel-like adhesion-preventing materials having a higher concentration and viscosity than before. Therefore, the material can be applied to a desired invasion site from a narrow port in laparoscopic surgery, and thus has excellent handling properties.

The adhesion-preventing material of the present invention is biodegradable, and is suitably absorbed and excreted from body tissues. Therefore, the material is highly biologically safe.

The adhesion-preventing material of the present invention is not particularly limited, as long as it comprises the above triblock copolymer (1) (hereinafter simply referred to as "the triblock copolymer") and the above polyethylene glycol (PEG) and/or methoxy-polyethylene glycol (2) (also referred to as "MeO-PEG" or "polyethylene glycol monomethyl ether"). Examples of the adhesion-preventing material of the present invention include (i) an adhesion-preventing material comprising only a triblock copolymer and PEG and/or MeO-PEG; (ii) an adhesion-preventing material further comprising a drug, in addition to the above (i); (iii) an adhesion-preventing material comprising a triblock copolymer, water, and PEG and/or MeO-PEG; (iv) an adhesion-preventing material further comprising a drug, in addition to the above (iii); and the like. In the following description, the present invention is described on the assumption that the adhesion-preventing material of the present invention is solid, unless otherwise specified; however, the adhesion-preventing material of the present invention is not limited to a solid form. That is, the adhesion-preventing material of the present invention includes solid, liquid, and gel forms.

Examples of the adhesion-preventing material of the present invention in a solid form include one obtained by simply adding the (solid) polyethylene glycol (2) to the triblock copolymer (1), one obtained by drying the adhesion-preventing material of the present invention in a liquid form dissolved in a solvent (a state after step 3), and the like.

Examples of the adhesion-preventing material of the present invention in a liquid form include one obtained by simply adding the (liquid) polyethylene glycol (2) to the triblock copolymer (1), one obtained by dissolving the triblock copolymer (1) and polyethylene glycol (2) in a solvent (a state after step 2), one obtained by dissolving the solid adhesion-preventing material of the present invention in water or a water-containing medium, and the like.

Examples of the adhesion-preventing material of the present invention in a gel form include one obtained by dissolving the solid adhesion-preventing material of the present invention in water or a water-containing medium, followed by heating, and the like.

### (1) Triblock Copolymer

The adhesion-preventing material of the present invention comprises an A-B-A type or B-A-B type triblock copolymer. The triblock copolymer (1) is biodegradable, and has an A segment containing poly (ε-caprolactone-co-glycolic acid) (PCGA), and a B segment containing polyethylene glycol (PEG).

There is no limitation on the physical properties of the triblock copolymer, such as the mass ratio of each of the PCGA segment (A segment) and the PEG segment (B segment) to the triblock copolymer, the weight average molecular weight of each of the PCGA and PEG segments, the number average molecular weight (Mn) of the triblock copolymer, the ratio of the weight average molecular weight to the number average molecular weight (Mw/Mn) of the triblock copolymer, the molar ratio of ε-caprolactone to glycolic acid in PCGA, and the polymerization degrees of ε-caprolactone and glycolic acid.

Preferred examples of the above physical properties are shown below. In the case of an A-B-A type triblock copolymer, the mass ratio of the PCGA and PEG segments is preferably such that the PCGA segment constitutes 30 to 75 mass% of the triblock copolymer, and the PEG segment constitutes 25 to 70 mass% of the triblock copolymer. The weight average molecular weight of the PCGA segment is preferably 1.0 k to 2.0 k (1,000 to 2,000). The weight average molecular weight of the PEG segment is preferably 1.0 k to 1.5 k (1,000 to 1,500). The number average molecular weight (Mn) of the triblock copolymer is preferably 3.0 k to 5.5 k (3,000 to 5,500). The ratio of the weight average molecular weight to the number average molecular weight (Mw/Mn) of the triblock copolymer is preferably 1.0 to 1.8. The molar ratio of ε-caprolactone (CL) to glycolic acid (GA) (ε-caprolactone:glycolic acid) in PCGA is preferably 7.5:1 to 9.5:1. The polymerization degree of ε-caprolactone is preferably 7.5 to 15, and the polymerization degree of glycolic acid is preferably 1.0 to 2.0.

In the case of a B-A-B type triblock copolymer, the mass ratio of the PCGA and PEG segments is preferably such that the PCGA segment constitutes 30 to 75 mass% of the triblock copolymer, and the PEG segment constitutes 25 to 70 mass% of the triblock copolymer. The weight average molecular weight of the PCGA segment is preferably 2.0 k to 3.0 k (2,000 to 3,000). The weight average molecular weight of the PEG segment is preferably 0.5 k to 1.0 k (500 to 1,000). The number average molecular weight (Mn) of the triblock copolymer is preferably 3.0 k to 5.0 k (3,000 to 5,000). The ratio of the weight average molecular weight to the number average molecular weight (Mw/Mn) of the triblock copolymer is preferably 1.0 to 1.8. The molar ratio of E-caprolactone to glycolic acid (ε-caprolactone:glycolic acid) in PCGA is preferably 7.5:1 to 9.5:1. The polymerization degree of ε-caprolactone is preferably 7.5 to 13, and the polymerization degree of glycolic acid is preferably 1.0 to 2.0.

The molar ratio, polymerization degree, and mass ratio can be measured by using a known method, such as ¹H-NMR. The number average molecular weight and weight average molecular weight can be measured by, for example, using GPC (Gel Permeation Chromatography), etc., in addition to ¹H-NMR mentioned above.

An A-B-A type triblock copolymer can be represented by the following formula (A):

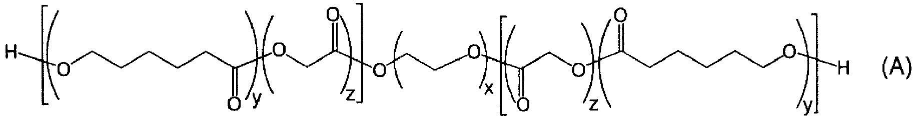

This triblock copolymer is also referred to as PCGA-b-PEG-b-PCGA (b means "block"). In formula (A), x, y, and z are values that can satisfy the above physical properties. For example, x is preferably a number of 20 to 40, and more preferably 22 to 35. y is preferably a number of 7.5 to 15, and more preferably 10 to 15. z is preferably a number of 1 to 3, and more preferably 1 to 2. Moreover, two ys in formula (A) may be the same number or different numbers. The same applies to two zs in formula (A). x, y, and z each represent the average number of units in the polymer, and are determined by ¹H-NMR and GPC. In the PCGA segment indicated by square brackets in formula (A), there is no rule in the sequence of ε-caprolactone units and glycolic acid units, and the sequence order is not limited to that of formula (A). Furthermore, y and z represent the average number of ε-caprolactone units and glycolic acid units, respectively, contained in one PCGA segment.

A B-A-B type triblock copolymer can be represented, for example, by the following formula (B): 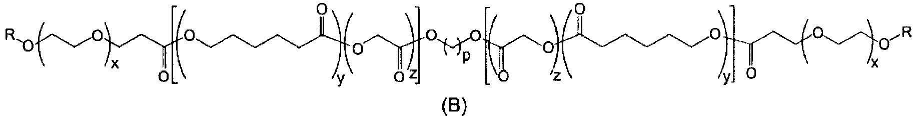 in formula (B), Rs are the same or different, and each represent H or CH₃; or by the following formula (C): 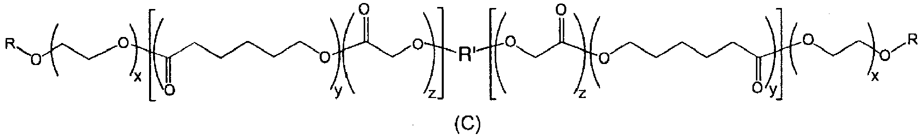 in formula (C), Rs are the same or different, and each represent H or CH₃, and R' represents the following:

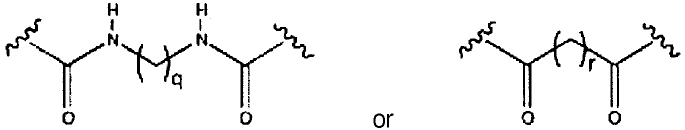

In formula (B) or (C), x, y, and z are also values that can satisfy the above physical properties. x is preferably a number of 10 to 25, and more preferably 11 to 23. y is preferably a number of 4 to 12, and more preferably 5 to 7. z is preferably a number of 0.5 to 2, and more preferably 1 to 2. Moreover, each pair of two xs, two ys, and two zs in formula (B) or (C) may be the same number or different numbers. p is preferably a number of 2 to 6, q is preferably a number of 1 to 6, and r is preferably a number of 1 to 6. x, y, and z each represent the average number of units in the polymer, and are determined by ¹H-NMR and GPC. In the PCGA segment indicated by square brackets in formula (B) or (C), there is no rule in the sequence of ε-caprolactone units and glycolic acid units, and the sequence order is not limited to that of formula (B) or (C). Furthermore, y and z each represent the average number of ε-caprolactone units and glycolic acid units, respectively, contained in one PCGA segment.

The triblock copolymer has a gel transition temperature between room temperature and body temperature. Specifically, when the triblock copolymer is dissolved in water or a water-containing medium, such as phosphate-buffered saline (PBS), the triblock copolymer is in a sol state at room temperature, and converted into a gel state when heated to around body temperature. The PCGA-b-PEG-b-PCGA is solid at ordinary temperature and in the absence of solvent, and has a sol-gel transition temperature of about 28°C.

The sol-gel transition temperature of the adhesion-preventing material of the present invention, the dynamic strength in a gel state, etc., can be adjusted by controlling the molecular weights of the PEG and PCGA segments in the copolymer, the content of PEG and/or MeO-PEG described later, the concentration of the triblock copolymer, and other factors.

An example of the production method (polymerization method) of the triblock copolymer is described later.

### (2) Polyethylene Glycol (PEG) and/or Methoxy-Polyethylene Glycol (MeO-PEG)

The adhesion-preventing material of the present invention comprises PEG and/or MeO-PEG, together with a triblock copolymer. Therefore, the obtained adhesion-preventing material can maintain the temperature-responsive sol-gel transition state, and can be dissolved in water or a water-containing medium for a short period of time without the need to be heated. These effects are presumably attributable to the following reasons. Because the solid triblock copolymer is adsorbed to water-soluble PEG and/or MeO-PEG on the molecular level, the adhesion-preventing material has:
(1) a property to maintain the temperature-responsive sol-gel transition state and solid state, derived from the triblock copolymer; and
(2) water solubility derived from PEG and/or MeO-PEG.
This PEG is one component contained in the adhesion-preventing material of the present invention, and is clearly differentiated from the PEG constituting the B segment of the triblock copolymer. The PEG and MeO-PEG may be used singly or in combination. When the PEG and MeO-PEG are used in combination, the ratio of PEG to MeO-PEG (PEG:MeO-PEG) is not particularly limited, but is preferably 99.9:0.1 to 0.1:99.9 (mass ratio).

The content of PEG and/or MeO-PEG is not particularly limited, but is preferably 2 to 20 parts by mass, and more preferably 5 to 20 parts by mass, based on 100 parts by mass of the triblock copolymer (1). When the content of PEG and/or MeO-PEG is within the above range, the adhesion-preventing material can be dissolved in water or a water-containing medium for a shorter period of time, and has excellent uniformity and fluidity after redissolution.

The weight average molecular weight of PEG and MeO-PEG is not particularly limited, but is preferably 750 to 50,000, and more preferably 1,000 to 10,000. When the weight average molecular weight of PEG and MeO-PEG is within the above range, the adhesion-preventing material can be dissolved in water or a water-containing medium for a shorter period of time, and has excellent uniformity and fluidity after redissolution.

In the adhesion-preventing material of the present invention, the concentration of the triblock copolymer at which the adhesion-preventing material shows temperature-responsive behavior is generally about 15 to 35 mass%, and preferably about 20 to the 35 mass%. The adhesion-preventing material of the present invention can be diluted with water or a water-containing medium, such as physiological saline, phosphate-buffered saline (PBS), or salt-free phosphate buffer. The present invention also includes such adhesion-preventing materials diluted with water or a water-containing medium.

When the liquid adhesion-preventing material of the present invention is produced to comprise 10 mass% of triblock copolymer, and immersed in a phosphate buffer with a pH of 7.4 at 37°C higher than the gelation temperature, the reduction rate of molecular weight after 13 days is preferably 70 to 80%.

The adhesion-preventing material of the present invention can be dried to a solid (powder) state. The drying method may be performed in the manner described below. The solid (powder) adhesion-preventing material of the present invention has excellent storage stability, and can be redissolved in water or a water-containing medium mentioned above for a short period of time.

The adhesion-preventing materials of the present invention may be used singly or in combination of two or more.

Moreover, other known biodegradable polymers, such as polylactic acid, polyglycolic acid, polycaprolactone (or a lactic acid/glycolic acid copolymer, a lactic acid/glycolic acid/ε-caprolactone copolymer), and gelatin, may be added to the adhesion-preventing material of the present invention within a range that does not impair the effects of the adhesion-preventing material of the present invention. The amount of such a polymer is 50 parts by mass or less, preferably 30 parts by mass or less, and more preferably 10 parts by mass or less, based on 100 parts by mass of the adhesion-preventing material of the present invention.

Application of a combination of two or more adhesion-preventing materials of the present invention or a combination of the adhesion-preventing material of the present invention with other known biodegradable polymers can not only improve the adhesion-preventing effect, but also reduce the dosage of the adhesion-preventing material.

The adhesion-preventing material of the present invention can be mixed with a known drug, and used as a pharmaceutical composition.

When applied in combination with other drugs, the adhesion-preventing material of the present invention may have the effects of, for example, reducing the side effects of the drugs and enhancing the therapeutic effects of the drugs.

When the adhesion-preventing material of the present invention is mixed with a drug, the handling during application is easy because the material is liquid at around room temperature. In contrast, the material is an insoluble gel at around body temperature; thus, the material becomes an insoluble substance after application, preventing early diffusion of the drug, and enhancing the retention of the drug in the administration site.

The drugs used in the pharmaceutical composition are not particularly limited, and can be healing-promoting drugs. Examples thereof include peptides having physiological activity, proteins, and other antibiotics; antitumor agents, antipyretics, analgesics, antiphlogistics, expectorant cough suppressants, sedatives, muscle relaxants, antiepileptic agents, antiulcer agents, antidepressants, anti-allergic agents, cardiotonics, antiarrhythmics, vasodilators, pressure-lowering diuretics, diabetes therapeutic agents, anticoagulants, hemostatics, tuberculostatic agents, hormonal agents, narcotic antagonists, and the like. These drugs may be used singly or in combination of two or more.

The drug content of the pharmaceutical composition of the present invention can be suitably selected depending on the type of drug etc. In the present invention, the drug content can be 0.00001 to 50 parts by mass, and preferably 0.0001 to 1 part by mass, based on 100 parts by mass of the adhesion-preventing material.

In particular, when the adhesion-preventing material of the present invention is used as a sustained-release injection, the drug content is determined depending on the type of drug, the sustained-release period, etc. For example, when a peptide is used as a drug to obtain an adhesion-preventing material showing sustained release for about one week to about one month, the peptide content may be generally about 10 mass% to 50 mass%, based on the total mass of the pharmaceutical composition.

The adhesion-preventing material of the present invention can be uniformly mixed with such drugs without inactivating the effects of the drugs. During the healing time of the invasion site, the adhesion-preventing material of the present invention remains in the invasion site, and sustainably releases the drug without inhibiting the effect of the drug. After healing, the adhesion-preventing material is safely absorbed and decomposed in the body.

More specifically, in consideration of the problem of infection, the adhesion-preventing material of the present invention can contain antimicrobial drugs. Preferred among such drugs regarding antibacterial properties are those having a wide antimicrobial spectrum.

The method for mixing the adhesion-preventing material of the present invention with such a drug is not particularly limited, and a known method can be used. For example, the adhesion-preventing material and a drug can simply be mixed; alternatively, a drug can be bound to each polymer, which is a component of the adhesion-preventing material, without losing the efficacy of the drug.

### 2. Method for Producing Adhesion-Preventing Material

The method for producing the adhesion-preventing material of the present invention is not particularly limited. For example, the production method sequentially comprises the following (i) and (ii):
(i) step 1 of polymerizing ε-caprolactone and glycolide in the presence of polyethylene glycol, methoxy-polyethylene glycol, or aliphatic diol, thereby producing the triblock copolymer (1); and
(ii) step 2 of dissolving the triblock copolymer (1) and the polyethylene glycol and/or methoxy-polyethylene glycol (2) in a solvent.
This production method can suitably produce an adhesion-preventing material comprising the above triblock copolymer (1) and PEG and/or MeO-PEG (2). The PEG or MeO-PEG in step 1 is a component used as a polymeric initiator and constituting the triblock copolymer (1). On the other hand, the PEG and/or MeO-PEG in step 2 is a component contained in the adhesion-preventing material of the present invention. Both components are clearly differentiated.

### Step 1

In step 1 of the production method of the present invention, a triblock copolymer is produced through the process of polymerizing ε-caprolactone and glycolide in the presence of PEG, MeO-PEG, or aliphatic diol.

An example of the polymerization method is shown below. The triblock copolymer of formula (A) mentioned above can be produced by polymerizing ε-caprolactone and glycolide in the presence of Sn(Oct)₂ (tin octylate) using PEG as a polymeric initiator. The triblock copolymer of formula (B) mentioned above can be produced by polymerizing ε-caprolactone and glycolide using an aliphatic diol as an initiator, and binding PEG and/or MeO-PEG to the terminal of the obtained symmetrical polymer. The triblock copolymer of formula (C) mentioned above can be produced by polymerizing ε-caprolactone and glycolide using MeO-PEG to thereby synthesize AB diblock copolymers, and binding the AB diblock copolymers together by a condensation reaction using a diisocyanate compound, dicarboxylic acid compound, or the like.

Examples of aliphatic diols include ethylene glycol, 1,2-propylene glycol, 1,3-propylene glycol, 1,4-butanediol, and the like.

The amounts of the starting materials (ε-caprolactone, glycolide, PEG, MeO-PEG, and aliphatic diol) are not particularly limited, and can be suitably adjusted, for example, so as to satisfy the above-mentioned preferred physical properties of the triblock copolymer. ε-caprolactone, glycolide, PEG, MeO-PEG, aliphatic diol, and Sn(Oct)₂ may be used in dry form.

After completion of the polymerization, reprecipitation is performed using good and poor solvents, and the precipitate is dried, thereby obtaining white powder of a triblock copolymer. The good solvent can be, for example, chloroform, and the poor solvent can be, for example, diethyl ether.

### Step 2

In step 2 of the production method of the present invention, the triblock copolymer (1) and PEG and/or MeO-PEG (2) are dissolved in a solvent. Thereby, the liquid adhesion-preventing material of the present invention in which the triblock copolymer and PEG and/or MeO-PEG are uniformly dissolved in the solvent is obtained.

The solvent is not particularly limited as long as it can dissolve the triblock copolymer and PEG and/or MeO-PEG. For example, water, a water-containing medium, acetone, or the like can be used. In particular, a water-containing medium is preferably used. The water-containing medium can be the abovementioned water-containing medium (i.e., physiological saline, phosphate-buffered saline, or salt-free phosphate buffer). As for the amount of the solvent, it is preferable to use the solvent so that the concentration of the triblock copolymer is about 20 to 35 mass%.

### Step 3

In the production method of the present invention, the liquid adhesion-preventing material of the present invention may be dried to a solid state in step 3 after step 2.

The drying method is not particularly limited. Examples thereof include freeze-drying, hot-air drying, vacuum decompression drying, infrared heating-drying, microwave heating-drying, and the like. Preferred among these is freeze-drying. When the liquid adhesion-preventing material of the present invention is freeze-dried, a solution containing the triblock copolymer and PEG and/or MeO-PEG is frozen, and the frozen solution is dried under vacuum pressure to sublimate the frozen solvent. The solid adhesion-preventing material of the present invention obtained by freeze-drying has a number of gaps and a large surface area. Therefore, this adhesion-preventing material has excellent storage stability, and is easily redissolved in water or a water-containing medium mentioned above for a shorter period of time.

The conditions of freeze-drying are not limited. For example, freezing is performed at a temperature that allows freezing (less than 0°C), followed by drying for 24 hours at a pressure of about 5 to 20 Pa at room temperature (e.g., 10 to 20°C). Thus, the solid adhesion-preventing material of the present invention is obtained.

To obtain the solid adhesion-preventing material of the present invention, it is preferable to dry the liquid adhesion-preventing material of the present invention comprising about 1 to 5 mass% of triblock copolymer by the above-mentioned drying method.

### 3. Method for Applying Adhesion-Preventing Material

The adhesion-preventing material of the present invention can be applied to body tissues of mammals including humans.

The application method is not particularly limited. A sol-like aqueous solution obtained by dissolving the solid (powder) adhesion-preventing material of the present invention in water or a water-containing medium is applied, for example, by coating, spraying, dipping, or the like to cover body tissues, such as organs.

The coating method can be a conventionally used method. For example, the coating method can be one in which the sol-like adhesion-preventing material is applied to body tissues with a syringe etc.

The spraying method can be a conventionally used method. Examples thereof include a dispenser spray equipped with a sprayable pump nozzle, trigger spray, aerosol spray, manual atomizer, power atomizer, and the like.

The dipping (immersion) method is not particularly limited. For example, the liquid adhesion-preventing material is poured in the abdominal cavity to cover organs etc., and then the extra liquid adhesion-preventing material is removed by suction or the like.

The adhesion-preventing material of the present invention can be applied not only in the form of a liquid (sol) aqueous solution, but also in a gel form. Further, a solid (powder) adhesion-preventing material can also be directly applied to body tissues.

A gel-like adhesion-preventing material can be easily obtained by, for example, adding water or a water-containing medium to the adhesion-preventing material of the present invention, followed by heating.

The obtained gel-like adhesion-preventing material can be applied to body tissues, for example, using a spatula, brush, etc.

Moreover, when a solid (powder) adhesion-preventing material is used, for example, it can be directly applied to body tissues. In this case, the solid (powder) adhesion-preventing material becomes a sol due to the moisture in the body, and then becomes a gel due to the body temperature.

The solid (powder) adhesion-preventing material can be applied to body tissues, for example, by rubbing the material using a spatula, brush, etc.

### 4. Properties of Adhesion-Preventing Material

The adhesion-preventing material of the present invention has excellent biocompatibility, biodegradability, and temperature-responsiveness, as described above.

The term "temperature-responsive sol-gel transition" as used herein generally refers to a property in which a solution containing the adhesion-preventing material undergoes a transition from a sol (liquid) state to a gel (solid) state at gelation temperature, which serves as a boundary. Specifically, this term refers to a property in which a sol (liquid) adhesion-preventing material becomes a gel when heated to a temperature equal to or higher than the gelation temperature, and when cooled to a temperature lower than the gelation temperature, the adhesion-preventing material is dissolved again and returns to a transparent sol state.

In the present specification, the gelation temperature of the aqueous solution of the adhesion-preventing material is determined by a dynamic viscoelasticity test as a temperature in which storage modulus G' exceeds loss modulus G". Moreover, the gelation temperature can be determined by measuring the viscosity change of the aqueous solution of the adhesion-preventing material by a test-tube inversion method.

The aqueous solution of the adhesion-preventing material of the present invention has a gelation temperature in the range of about 10 to 50°C. The gelation temperature can be easily adjusted in this range, and its application range is very wide. For example, in the case of an aqueous solution of the adhesion-preventing material of the present invention having a gelation temperature of 25 to 35°C, the gelation temperature is present between room temperature (e.g., about 10 to 20°C) and body temperature (about 35 to 37°C). Accordingly, the adhesion-preventing material can be administered into the body in a sol (liquid) state, and hydrogel can be formed in the body.

In order for the gel-like adhesion-preventing material to prevent adhesion of body tissues, it is desirable that the adhesion-preventing material is generally present in the affected area (invasion site) over about one month, and preferably about two weeks.

Because the adhesion-preventing material of the present invention is a liquid state at around room temperature, when, for example, the material is applied (coated) to body tissues using a syringe etc., the handling is easy. On the other hand, because the material becomes an insoluble gel at around body temperature, the material becomes an insoluble substance after it is applied into the body; therefore, the retention of the adhesion-preventing material in body tissues can be improved.

The adhesion-preventing material of the present invention can be used for other medical materials.

### Advantageous Effects of Invention

The adhesion-preventing material of the present invention undergoes a sol-gel transition in response to temperature. When the adhesion-preventing material of the present invention is used, heating is not required, and the dissolving time in water etc. is short. Therefore, the adhesion-preventing material of the present invention can be easily prepared before use. Moreover, in contrast to film-like (sheet-like) adhesion-preventing materials, the adhesion-preventing material of the present invention is in the form of an aqueous solution when used, and it can therefore be easily applied to invasion sites having a complicated shape or structure. After application, the adhesion-preventing material of the present invention becomes a gel due to body temperature; therefore, the material can stably exhibit an adhesion-preventing effect for a desired period of time. The adhesion-preventing material of the present invention can be applied to a desired invasion site from a narrow port in laparoscopic surgery, and thus has excellent handling properties. In addition, since the adhesion-preventing material is properly absorbed and excreted from body tissues, it is highly biologically safe.

### Brief Description of Drawings

Fig. 1 is a graph showing the storage modulus (G') and loss modulus (G") of the adhesion-preventing materials (aqueous solutions) with respect to temperatures obtained by rheometer measurement in Test Example 3.
Fig. 2 shows photographs of the adhesion-preventing material of Example 10 in the following states: (a) a solidified state after vacuum-freezing, (b) a state immediately after PBS was added to the material in state (a) above, (c) a state after the material in state (b) above was stirred with a vortex mixer, and (d) a state after the material in state (c) above was maintained at 37°C.
Fig. 3 is a graph showing the storage modulus (G') and loss modulus (G") of the adhesion-preventing materials (aqueous solutions) with respect to temperatures obtained by rheometer measurement in Test Example 4.

### Description of Embodiments

The present invention is described in detail below with reference to Examples. However, the present invention is not limited to the Examples.

### Production Example 1

### Synthesis of Triblock Copolymer

PEG (1,540) (polyethylene glycol (1,540), produced by Wako Pure Chemical Industries, Ltd.) alone was dried for 3 hours while applying heat with a dryer. Then, ε-caprolactone (produced by Wako Pure Chemical Industries, Ltd.), glycolide (produced by Polysciences, Inc.), and Sn(Oct)₂ (produced by Wako Pure Chemical Industries, Ltd.) were added thereto, and the mixture was further dried for 6 hours. Subsequently, polymerization was performed in an oil bath at 160°C for 12 hours. After completion of the reaction, reprecipitation was performed using chloroform as a good solvent and diethyl ether as a poor solvent, thereby obtaining a white precipitate. After the precipitate was dried, white powder of a PCGA-b-PEG-b-PCGA triblock copolymer (polymer) was obtained. Table 1 below shows the physical properties of the triblock copolymer. The melting point (Tm) of the triblock copolymer (1) was 33°C.

**Table 1**

| Code | Mn^{a)} | Mw/Mn ^{b)} | CA/GA^{a)} (mol/mol) | DP of CA^{a,c)} | DP of GA^{a,d)} | PEG content (wt%)^{a)} | solubility in water |
|---|---|---|---|---|---|---|---|
| 1.7k-1.5k-1.7k | 5,000 | 1.6 | 9.0 | 14 | 1.5 | 30.6 | good |

| | | | | | | | |
|---|---|---|---|---|---|---|---|
| a) Measured by ¹H-NMR (solvent: CDCl₃) b) Measured by GPC (eluent: DMF, standard: PEG, Tosoh GPC-8020 series system produced by Tosoh Co.) c) Degree of polymerization of ε-caprolactone d) Degree of polymerization of glycolide | | | | | | | |

When phosphate-buffered saline (PBS) (pH = 7.4) was added to the triblock copolymer white powder so that the mass% of the triblock copolymer was 30 wt%, the triblock copolymer was not dissolved only by adding the PBS. When heat was applied with a dryer, followed by cooling, the triblock copolymer was dissolved in the PBS.

### Example 1

### Production of Adhesion-Preventing Material

The triblock copolymer (100 mg) obtained in Production Example 1 and 5 mg of PEG (1,000) were weighed, and placed in a sample tube. After 1 mL of acetone was further added to the sample tube, ultrasonic treatment was performed using a sonicator, thereby obtaining a solution in which the triblock copolymer and PEG were dissolved in the acetone. The solution was then added dropwise to an eggplant-shaped flask containing 9.895 mL of ultrapure water, and stirred for 20 minutes. Subsequently, the acetone was removed by an evaporator, thereby obtaining a liquid adhesion-preventing material (triblock copolymer concentration: 1 wt%) of Example 1.

Next, the liquid adhesion-preventing material was freeze-dried under vacuum. Specifically, the liquid adhesion-preventing material was frozen with liquid nitrogen, and dried at a pressure of about 5 to 20 Pa at room temperature (20°C) for 24 hours. Thus, a solid adhesion-preventing material of Example 1 was obtained.

### Examples 2 to 20 and Comparative Examples 1 to 5

Liquid adhesion-preventing materials (triblock copolymer concentration: 1 wt%) and solid adhesion-preventing materials were obtained in the same manner as in Example 1, except that the type, amount, etc., of added substances were suitably changed as shown in Table 3. In Examples 13 to 19 and Comparative Examples 1 to 4, the above solution was obtained using a mixture of 1 mL of acetone and 1 mL of pure water in place of 1 mL of acetone, and the solution was added dropwise to an eggplant-shaped flask containing 8.895 mL of ultrapure water in place of 9.895 mL of ultrapure water. Other conditions were the same as those of Example 1. HANa of Comparative Example 2 represents sodium hyaluronate, PAA of Comparative Example 3 represents polyacrylic acid, and PVA of Comparative Example 4 represents polyvinyl alcohol. The details of the added substances are shown below.
- PEG (1,000): polyethylene glycol (1,000), produced by Wako Pure Chemical Industries, Ltd.
- PEG (1,540): polyethylene glycol (1,540), produced by Wako Pure Chemical Industries, Ltd.
- PEG (2,000): polyethylene glycol (2,000), produced by Sigma-Aldrich Co., LLC
- PEG (4,600): polyethylene glycol (4,600), produced by Sigma-Aldrich Co., LLC
- PEG (10,000): polyethylene glycol (10,000), produced by Sigma-Aldrich Co., LLC
- MeO-PEG: polyethylene glycol (5,000) monomethyl ether, produced by Sigma-Aldrich Co., LLC
- Sucrose: sucrose (saccharose), produced by Wako Pure Chemical Industries, Ltd.
- HANa: sodium hyaluronate (90,000), produced by Kibun Food Chemifa Co., Ltd.
- PAA: polyacrylic acid (5,000), produced by Wako Pure Chemical Industries, Ltd.
- PVA: polyvinyl alcohol (2,000), produced by Kanto Chemical Co., Inc.

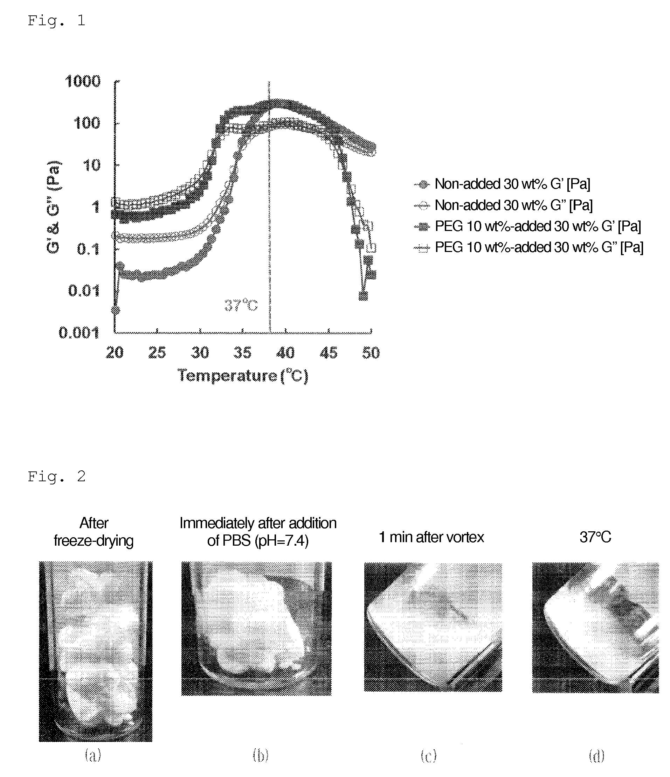

Fig. 2 shows photographs of the adhesion-preventing material of Example 10 in the following states: (a) a solidified state after vacuum freezing, (b) a state immediately after PBS was added to the material in state (a) above, (c) a state after the material in state (b) above was stirred with a vortex mixer, and (d) a state after the material in state (c) above was maintained at 37°C.

### Example 21

A triblock copolymer (melting point Tm = 33°C) having the physical properties shown in Table 2 below was obtained in the same manner as in Production Example 1.

**Table 2**

| Code | Mn^{a)} | Mw/Mn ^{b)} | CA/GA^{a)} (mol/mol) | DP of CA^{a,c)} | DP of GA^{a,d)} | PEG content (wt%)^{a)} | solubility in water |
|---|---|---|---|---|---|---|---|
| 1.8k-1.5k-1.8k | 5,100 | 1.6 | 8.1 | 14 | 1.7 | 30.2 | good |

| | | | | | | | |
|---|---|---|---|---|---|---|---|
| a) Measured by ¹H-NMR (solvent: CDCl₃) b) Measured by GPC (eluent: DMF, standard: PEG, Tosoh GPC-8020 series system produced by Tosoh Co.) c) Degree of polymerization of ε-caprolactone d) Degree of polymerization of glycolide | | | | | | | |

The above triblock copolymer (150 mg) and 15 mg of PEG (2,000) were weighed, and placed in a sample tube. After 1.5 mL of acetone was further added to the sample tube, ultrasonic treatment was performed using a sonicator, thereby obtaining a solution in which the triblock copolymer and PEG were dissolved in the acetone. The solution was then added dropwise to an eggplant-shaped flask containing 14.835 mL of ultrapure water, and stirred for 20 minutes. Subsequently, the acetone was removed by an evaporator, thereby obtaining a liquid adhesion-preventing material (triblock copolymer concentration: 1 wt%) of Example 21.

Next, the liquid adhesion-preventing material was freeze-dried under vacuum. Specifically, the liquid adhesion-preventing material was frozen with liquid nitrogen, and dried at a pressure of about 5 to 20 Pa at room temperature (20°C) for 24 hours. Thus, a solid adhesion-preventing material of Example 21 was obtained.

### Comparative Example 6

An adhesion-preventing material was obtained in the same manner as in Example 21, except that PEG (2,000) was not used.

### Comparative Example 7

An adhesion-preventing material was obtained in the same manner as in Example 21, except that 15 mg of sucrose was used in place of 15 mg of PEG (2,000).

### Test Example 1

### Redissolvability Test

PBS (pH = 7.4) was added to each of the solid adhesion-preventing materials of Examples 1 to 20 and Comparative Examples 1 to 5 so that the triblock copolymer concentration was 30 wt%, and the mixture was stirred with a vortex mixer at ordinary temperature. Then, whether each solid adhesion-preventing material was dissolved in the PBS was visually observed. Further, when the solid adhesion-preventing material was dissolved in the PBS, (i) the time required for the dissolution was measured, and (ii) the uniformity and fluidity of the dissolution were evaluated. Regarding (i) the time required for the dissolution, 3 minutes or less was regarded as acceptable. Regarding (ii), the uniformity of the dissolution was evaluated by visual observation, and the fluidity was evaluated based on whether the adhesion-preventing material dissolved in the PBS could pass through a syringe (inner diameter: 0.9 mm). The results were evaluated as follows:
Uniformly dissolved and fluid: "+"
Not uniformly dissolved and non-fluid: "-"
Table 3 below shows the test results.

### Test Example 2

### Temperature-Responsive Behavior Test

A PBS solution was added to each of the solid adhesion-preventing materials of Examples 1 to 20 and Comparative Examples 1 to 5 so that the triblock copolymer concentration was 30 wt%. The presence of sol-gel transition of each solution was examined by a test-tube inversion method.

Specifically, after each solution stored in a sample tube (diameter: about 1 cm) was heated to 37°C, the sample tube was inverted. When the solution did not flow for 30 seconds, it was regarded as gelled. The results were evaluated as follows:
Gelled at 37°C: "+"
Not gelled at 37°C: "-"
Table 3 below shows the test results.

**Table 3**

| | Added substance | Content of added substance (parts by mass based on 100 parts by mass of triblock copolymer) | Uniformity and fluidity during redissolution (dissolution in PBS) | Time required for dissolution during redissolution (dissolution in PBS) (min) | Presence of sol-gel transition (37°C) |
|---|---|---|---|---|---|
| Example 1 | PEG (1,000) | 5 | + | 2.0 | + |
| Example 2 | PEG (1,000) | 10 | + | 3.0 | + |
| Example 3 | PEG (1,000) | 15 | + | 2.0 | + |
| Example 4 | PEG (1,000) | 20 | + | 2.0 | + |
| Example 5 | PEG (1,540) | 5 | + | 1.0 | + |
| Example 6 | PEG (1,540) | 10 | + | 2.5 | + |
| Example 7 | PEG (1,540) | 15 | + | 2.0 | + |
| Example 8 | PEG (1,540) | 20 | + | 2.0 | + |
| Example 9 | PEG (2,000) | 7.5 | + | 2.0 | + |
| Example 10 | PEG (2,000) | 10 | + | 1.0 | + |
| Example 11 | PEG (2,000) | 12.5 | + | 1.0 | + |
| Example 12 | PEG (2,000) | 15 | + | 1.0 | + |
| Example 13 | PEG (4,600) | 5 | + | 1.5 | + |
| Example 14 | PEG (4,600) | 10 | + | 0.5 | + |
| Example 15 | PEG (4,600) | 15 | + | 2.0 | + |
| Example 16 | PEG (4,600) | 20 | + | 2.0 | + |
| Example 17 | PEG (10,000) | 5 | + | 1.5 | + |
| Example 18 | PEG (10,000) | 10 | + | 1.0 | + |
| Example 19 | PEG (10,000) | 15 | + | 2.0 | + |
| Example 20 | MeO-PEG (5,000) | 10 | + | 0.5 | + |
| Comparative Example 1 | Sucrose | 10 | + | 4.0 | + |
| Comparative Example 2 | HANa (90,000) | 10 | - | (*1) | (*2) |
| Comparative Example 3 | PAA | 10 | + | 3.5 | + |
| Comparative Example 4 | PVA | 10 | - | (*1) | (*2) |
| Comparative Example 5 | Not added | | + | (*3) | + |

| | | | | | |
|---|---|---|---|---|---|
| *1: The time required for dissolution could not be measured because the solid adhesion-preventing material was not dissolved in PBS. *2: The presence of sol-gel transition could not be examined because the solid adhesion-preventing material was not dissolved in PBS. *3: The solid adhesion-preventing material was not dissolved simply by stirring for 5 minutes or more. A uniform solution was only obtained by stirring for 5 minutes or more, and physically pulverizing the aggregate. | | | | | |

It was confirmed that the solid adhesion-preventing materials of Examples 1 to 20 were uniformly dissolved in the PBS within 3 minutes and were fluid. Moreover, the liquid adhesion-preventing materials of Examples 1 to 20 obtained by dissolution in the PBS were gelled at 37°C. In contrast, the solid adhesion-preventing materials of Comparative Examples 1, 3, and 5 were uniformly dissolved in the PBS; however, the time required for the dissolution exceeded 3 minutes. Moreover, the solid adhesion-preventing materials of Comparative Examples 2 and 4 were not uniformly dissolved in the PBS.

### Test Example 3

### Rheometer Measurement

PBS (pH = 7.4) was added to each of the solid adhesion-preventing materials of Example 21 (adhesion-preventing material containing 10 parts by mass of PEG based on 100 parts by mass of triblock copolymer) and Comparative Example 6 so that the triblock copolymer concentration was 30 wt%. The resulting mixtures were each stirred with a vortex mixer at ordinary temperature, thereby obtaining liquid adhesion-preventing materials.

The obtained liquid adhesion-preventing materials were each subjected to rheometer measurement to confirm the temperature-responsive sol-gel transition behavior. Fig. 1 and Table 4 show the test results.

**Table 4**

| | Condition | Gelation temperature | G' at 37°C (storage modulus) | G' MAX and its temperature |
|---|---|---|---|---|
| Comparative Example 6 | PEG-free (not added) | 34.4°C | 225 Pa | 308 Pa (38.9°C) |
| Example 21 | Containing 10 parts by mass of PEG based on 100 parts by mass of triblock copolymer | 31.9°C | 268 Pa | 304 Pa (39.4°C) |

The results of Test Example 3 confirmed that when PEG was contained (added), the gelation temperature was reduced by about 2.5°C.

### Test Example 4

### Rheometer Measurement

PBS (pH = 7.4) was added to each of the solid adhesion-preventing materials of Example 21, and Comparative Examples 6 and 7 so that the triblock copolymer concentration was 30 wt%. The resulting mixtures were each stirred with a vortex mixer at ordinary temperature, thereby obtaining liquid adhesion-preventing materials.

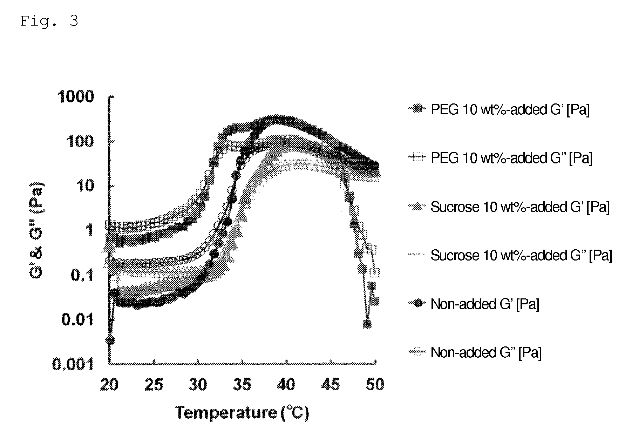

The obtained liquid adhesion-preventing materials were subjected to rheometer measurement to confirm the temperature-responsive sol-gel transition behavior. Fig. 3 and Table 5 show the test results.

**Table 5**

| | Condition | Gelation temperature | G' at 37°C (storage modulus) |
|---|---|---|---|
| Comparative Example 6 | PEG-free (not added) | 34.4°C | 225 Pa |
| Example 21 | Containing 10 parts by mass of PEG (2,000) based on 100 parts by mass of triblock copolymer | 31.9°C | 268 Pa |
| Comparative Example 7 | Containing 10 parts by mass of sucrose based on 100 parts by mass of triblock copolymer | 36.4°C | 29 Pa |

The solid adhesion-preventing material containing sucrose (Comparative Example 7) was viscous and not partially dissolved in the PBS. Further, the results of Test Example 4 showed that G' of the liquid adhesion-preventing material containing sucrose (Comparative Example 7) at 37°C was reduced. In contrast, the solid adhesion-preventing material containing PEG (Example 21) was non-viscous powder, and became a white emulsion in the PBS, in which no insoluble substance was observed, and the material was uniformly dissolved. The PBS solution in which the triblock copolymer was uniformly dissolved passed through a syringe without resistance when it was sucked by the syringe. Further, the results of Test Example 4 showed that G' of the liquid adhesion-preventing material containing PEG (Example 21) at 37°C was not reduced. These results confirmed that PEG was superior to sucrose as the added substance contained together with the triblock copolymer.

### Test Example 5

### Animal Experiment and Evaluation

Intraperitoneal adhesion rat models described below were used to evaluate the adhesion-preventing effect of the adhesion-preventing material of the present invention after it was applied for 14 days. In the evaluation of the adhesion-preventing material, the liquid adhesion-preventing material of Example 10 (prepared using PBS so that the polymer concentration was 10 wt%) was used as a test substance. Rat models for which the test substance was used were classified into "the group treated with the adhesion-preventing material of the present invention," and rat models for which the test substance was not used were classified into "the untreated group." Five models were used in each group.

First, the rats were intraperitoneally administered with sodium pentobarbital (30 mg/kg), and given a general anesthetic. After the abdomen of each rat was shaved, the abdomen was opened by a 3 or 4-cm midline abdominal incision, and the appendix was exposed from the wound. A certain area (1 to 2 cm²) of the exposed appendix on the small intestine side was scratched with sterile gauze until petechial hemorrhage occurred, and the scratch site was exposed to the air for 10 minutes. Thereafter, the test substance (coating amount: 100 µL) was applied to the scratch site, and the appendix was returned into the abdominal cavity. Then, the abdominal wall of the incision was sutured in two layers to close the abdominal cavity. After the wound was sterilized with Isodine (registered trademark) antiseptic, the rat was returned to the cage. On the 14th day after the model was created, the abdomen of the animal was reopened under sodium pentobarbital anesthesia, and the degree of adhesion in the adhesion-induced site was observed with the naked eye. Then, the degree (grade) of adhesion in the abdominal cavity was scored according to the following criteria.

### Grade Classification

Grade 0 (score 0): no adhesion observed
Grade 1 (score 1): easily separable thin adhesion
Grade 2 (score 2): weak adhesion in narrow area, but able to withstand slight traction
Grade 3 (score 3): remarkably strong adhesion observed, or adhesion observed in at least two sites
Grade 4 (score 4): adhesion observed in three or more sites

Table 6 below shows the test results of the group treated with the adhesion-preventing material of Example 10 of the present invention.

**Table 6**

| Sample No. | Adhesion prevention score |
|---|---|
| 1 | 0 |
| 2 | 0 |
| 3 | 0 |
| 4 | 2 (weak adhesion in one site between appendix and fat, easily removable) |
| 5 | 2 (weak adhesion in one site between appendix and fat) |

Table 7 below shows the test results of the untreated group.

**Table 7**

| Sample No. | Adhesion prevention score |
|---|---|
| 1 | 2 (strong adhesion in one site between appendix and fat) |
| 2 | 3 (strong adhesion in two sites between appendix and fat) |
| 3 | 2 (strong adhesion in one site only in appendix) |
| 4 | 0 |
| 5 | 0 |

The results of Test Example 5 showed that a smaller number of rats underwent adhesion in the group treated with the adhesion-preventing material of the present invention than in the untreated group. Furthermore, in view of the adhesion prevention score, one sample in the untreated group was given a Grade 3 score (score 3) in which the degree of adhesion was relatively higher, whereas no sample in the group treated with the adhesion-preventing material of the present invention was given a Grade 3 score (score 3).

## Claims

1. An adhesion-preventing material comprising the following (1) and (2):
(1) an A-B-A type or B-A-B type triblock copolymer that has an A segment containing poly(ε-caprolactone-co-glycolic acid) and a B segment containing polyethylene glycol; and
(2) polyethylene glycol and/or methoxy-polyethylene glycol.

2. The adhesion-preventing material according to claim 1, wherein the polyethylene glycol and/or methoxy-polyethylene glycol (2) is contained in an amount of 2 to 20 parts by mass based on 100 parts by mass of the triblock copolymer (1).

3. The adhesion-preventing material according to claim 1 or 2, wherein the A segment constitutes 30 to 75 mass% of the triblock copolymer (1), and the B segment constitutes 25 to 70 mass% of the triblock copolymer (1).

4. A method for producing an adhesion-preventing material comprising the following (1) and (2):
(1) an A-B-A type or B-A-B type triblock copolymer that has an A segment containing poly(ε-caprolactone-co-glycolic acid) and a B segment containing polyethylene glycol; and
(2) polyethylene glycol and/or methoxy-polyethylene glycol;
the method sequentially comprising the following (i) and (ii):
(i) step 1 of polymerizing ε-caprolactone and glycolide in the presence of polyethylene glycol, methoxy-polyethylene glycol, or aliphatic diol to produce the triblock copolymer (1); and
(ii) step 2 of dissolving the triblock copolymer (1) and the polyethylene glycol and/or methoxy-polyethylene glycol (2) in a solvent.

5. The method for producing an adhesion-preventing material according to claim 4, the method further comprising step 3 of drying the adhesion-preventing material obtained in step 2 to a solid state.
